# EUROPEAN PATENT SPECIFICATION

(11) **EP 0 920 843 B1**
(45) Date of publication and mention of the grant of the patent: **09.02.2005**
(21) Application number: 99101203.0
(22) Date of filing: 27.12.1991
(51) Int. Cl.: A61F 2/06, A61L 29/00, A61M 29/02

(54) **Drug delivery system**
Arzneistoffabgabesystem
Système d'administration de médicament

(30) Priority: 28.12.1990 US 635732; 22.11.1991 US 795976
(43) Date of publication of application: 09.06.1999
(62) Divisional of application: 92903283.7
(73) Proprietor: BOSTON SCIENTIFIC CORPORATION, Natick, MA 01760-1537 (US)
(72) Inventor: Sahatjian, Roland, Lexington, MA 02173 (US)
(74) Representative: Wallace, Sheila Jane

(56) References cited:
- EP-A- 0 357 003
- EP-A- 0 364 787
- EP-A- 0 372 088

## Description

### Field of the Invention

The invention relates to delivery of drugs to the walls of body lumens.

### Background of the Invention

systemic administration of drugs treats the organism as a whole, even though the disease may be localized, such as occlusion of a duct or vessel. Localization of a drug poses special problems in cases involving the walls of ducts and vessels, since, by nature, these organs serve as transport systems.

Atherosclerotic disease, for example, causes localized occlusion of the blood vessels resulting from the build-up of plaque. As the deposits increase in size, they reduce the diameter of the arteries and impede blood circulation. Angioplasty, which involves the insertion of catheters, such as balloon catheters, through the occluded region of the blood vessel in order to expand it, has been used to treat atherosclerosis.

The aftermath of angioplasty in many cases is problematic, due to restenosis, or closing of the vessel, that can occur from causes including mechanical abrasion and the proliferation of smooth muscle cells stimulated by the angioplasty treatment. Restenosis may also occur as a result of clot formation following angioplasty, due to injury to the vessel wall which triggers the natural clot-forming reactions of the blood.

EP-A-0357003 discloses a radially expandable endoprosthesis or stent which includes a plurality of adjacent generally circumferential sections that are substantially axially positioned with respect to each other. At least one of the generally circumferential sections has a generally circumferentially disposed expandable segment that imparts circumferential and radial expandability to the stent. The stent may be made out of a variety of materials and it is also possible to coat these materials with non-thrombogenic agents such as pyrolytic carbon, heparin, hydrogels, Teflon materials, silicones, polyurethanes and the like. The stent can also be treated so that drugs can be eluted therefrom.

EP-A-0364787 describes a plurality of expandable and deformable intraluminal vascular grafts which are expanded within a blood vessel by an angioplasty balloon associated with a catheter to dilate and expand the lumen of a blood vessel. The grafts may have a biologically compatible coating made of an absorbable polymer. Suitable absorbable polymers include polyglycolides, polylactides, and copolymers thereof. Such absorbable polymers may also contain various types of drugs whereby, as the coating is absorbed or dissolves, the drug is slowty released into the body passageway where the graft is located.

### Summary of the Invention

In one aspect, the invention features an endoprosthesis for delivering drug to tissue at a desired location of the wall of the body lumen. The endoprosthesis may be mounted on a catheter comprising a balloon. The catheter is constructed for insertion in a body lumen and has a catheter shaft and an expandable portion mounted on the catheter shaft. The expandable portion is expandable to a controlled pressure to fill the cross-section of the body lumen and press against the wall of the body lumen. At least a portion of the exterior surface of the expandable portion is defined by a coating of a tenaciously adhered swellable hydrogel polymer. Incorporated in the hydrogel polymer is an aqueous solution of a preselected drug to be delivered to the tissue. The hydrogel polymer and drug are selected to allow rapid release of a desired dosage of the drug from the hydrogel polymer coating during compression of the hydrogel polymer coating against the wall of the lumen when the expandable portion is expanded.

Various embodiments may include one or more of the following features. The catheter is adapted for insertion in a blood vessel, and the expandable portion is an inflatable dilatation balloon adapted for inflation at pressures in the range for effecting widening of a stenosed blood vessel. The pressure is in the range of 101 to 2027 kPa (1 to 20 atmospheres). The hydrogel polymer and drug are effective to release about 20% or more of the drug during inflation in the pressure range. The compression is effective to deliver the drug over a duration of about 10 minutes or less. The hydrogel polymer coating is about 10 to 50µm (microns) thick in the swelled, uncompressed state. The hydrogel polymer is selected from the group consisting of polycarboxylic acids, cellulosic polymers, gelatin, polyvinylpyrrolidone, maleic anhydride polymers, polyamides, polyvinyl alcohols, and polyethylene oxides. The hydrogel polymer is polyacrylic acid. The drug is an anti-thrombogenic drug selected from the group consisting of heparin, Pebac, enoxaprin, aspirin and hirudin. The drug is an anti-proliferative drug selected from the group consisting of monoclonal antibodies, capable of blocking smooth muscle cell proliferation, heparin, angiopeptin and enoxaprin. The expandable portion is adapted for application of heat to the polymer material to control the rate of administration. The catheter further comprises a sheath member, extendable over the balloon to inhibit release of the drug into body fluids during placement of the catheter. The balloon catheter is a perfusion catheter having an expandable balloon. The expandable portion includes a stent, mountable in the blood vessel by expansion thereof. The drug is bound in the hydrogel polymer for slow time release of the drug after the compression of the hydrogel polymer by the expansion. The hydrogel polymer is a polyacrylic acid including an ammonium cation and the drug is heparin. The stent is expandable by a balloon. The catheter where the stent and balloon both include the swellable hydrogel coating incorporating the drug. The expandable portion is prepared by introducing an aqueous solution of the drug to the hydrogel polymer coating, the catheter is introduced to the body lumen to position the expandable portion at the point of desired drug application, and the expandable portion is expanded to enable delivery of the drug by compression of the hydrogel polymer coating against the wall at the body lumen. The expandable portion is positioned at a point of occlusion in the blood vessel and expanding the expandable portion at pressures sufficient to simultaneously dilate the vessel and deliver the drug by compression of the hydrogel polymer coating.

The polymer is selected from the group consisting of polycaprolactone, polyorthoesters, polylactic acids, polyglycolic acids, and albumin. The catheter where the expandable portion is adapted for application of heat to the polymer material to control the rate of administration. The catheter where the polymer is a meltable polymer, and the release of the polymer is aided by the application of heat.

In general, an advantage of the invention is the application of drugs by active diffusion directly into the tissue within the body requiring treatment. The drug is preferably applied in a rapid but low-stress, low energy manner that does not further injure the tissue to be treated, and administration is selectively and evenly distributed over the treated area such that the drug can be taken up by tissue and plaque, without, e.g., being washed away by body fluids.

### Description of Preferred Embodiments

We first briefly describe the drawings.

### Drawings

Figs. 1-1a are enlarged views of a method of preparing a drug delivery balloon catheter.
Fig. 1b is an enlarged cross-sectional view of a drug delivery balloon catheter being moved through a vessel toward an occlusion to be treated.
Fig. 1c is an enlarged cross-sectional view of the balloon in Fig. 1b, now fully inflated and at the site of occlusion.
Fig. 1d is a further enlarged, schematic cross-sectional view of the portion of Fig. 1c indicated in the circle 1e, but taken prior to full inflation.
Fig. 1e, which corresponds to the portion of Fig. 1c indicated in the circle 1e, is an enlarged, schematic cross-sectional view, as in Fig. 1d, but with the balloon under full inflation to release the drug coated on the balloon.
Fig. 2 is an enlarged cross-sectional view of another embodiment of a drug delivery balloon catheter including a sheath for covering the catheter as it is being moved through a vessel toward the occlusion to be treated.
Fig. 2a is an enlarged cross-sectional view of the catheter of Fig. 2 with the sheath retracted and balloon inflated at the site of occlusion.
Fig. 3 is an enlarged, schematic cross-sectional view of another embodiment of a drug delivery balloon catheter in which the drug, originally held within a polymer applied to a thermal balloon, is now entering the surrounding tissue.
Fig. 3a is a further enlarged, schematic illustration of the embodiment of Fig. 3 and illustrates the entry of the drug, shown as circles, into the surrounding tissue.
Figure 4 shows a balloon catheter with the hydrogel and drug coated endoprosthesis of the invention mounted on the balloon section, in the region of the thrombus, before radial expansion of the balloon section and endoprosthesis.
Figure 4a is an enlargement of Figure 4 showing the hydrogel polymer and drug coated endoprosthesis and Fig. 4b is a cross-section along the line b-b in Fig. 4a.
Figure 5 shows the endoprosthesis compressed against the vessel wall by radial expansion of the balloon section with the drug diffused into the compressed thrombus before removal of the balloon catheter.
Figure 6 shows the endoprosthesis positioned against the drug inside the compressed thrombus, after removal of the balloon catheter.

### General Description

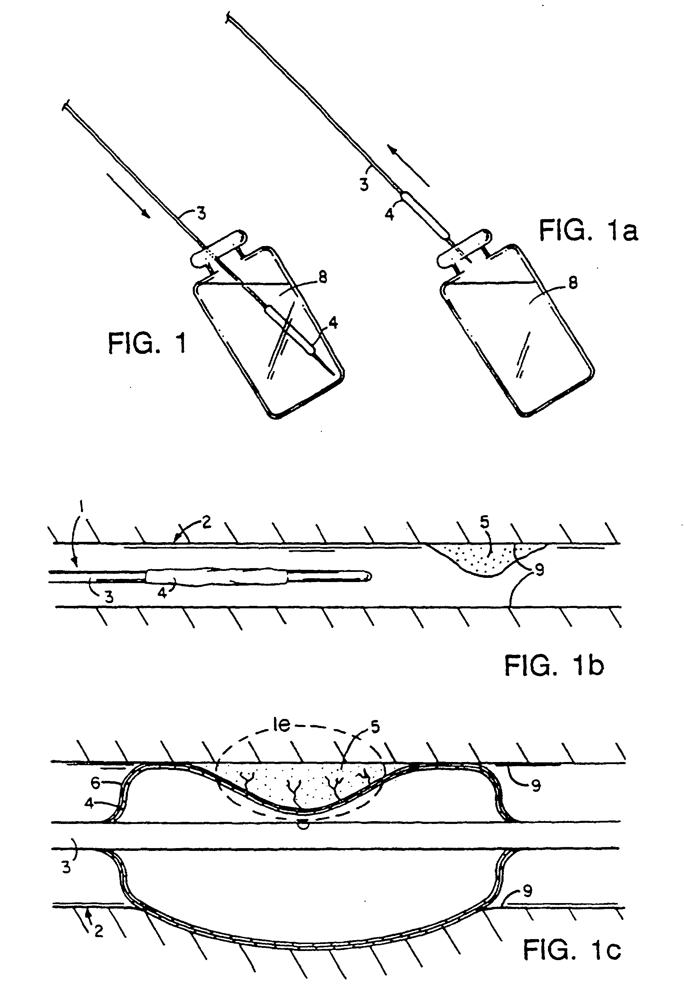

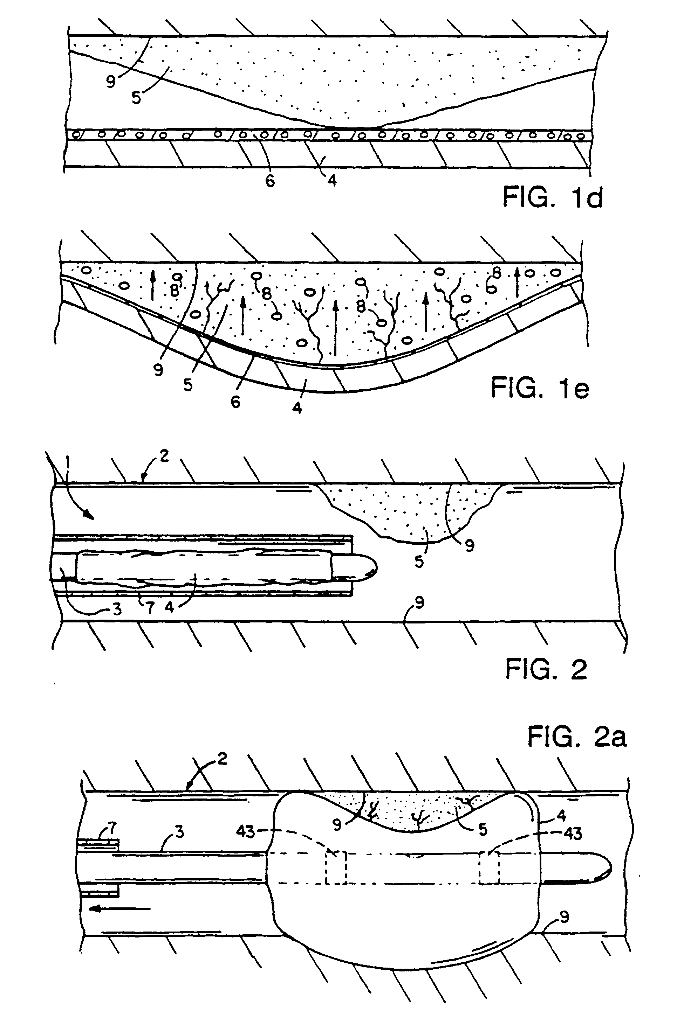

Referring to Figs. 1-1e,a drug delivery balloon catheter device 1 comprises a catheter body 3 having a balloon 4 attached at its distal end. The balloon 4 on the catheter 3 includes a swellable hydrogel polymer coating 6. As shown in Figs. 1-1a, a drug 8 in an aqueous solution is absorbed into the hydrogel with the balloon in the deflated state prior to insertion into the patient by the physician, e.g., the hydrogel-coated balloon may be immersed in a small tube or vial containing the drug. The drug may also be applied in the form of droplets, such as from an eyedropper, or the drug may be precipitated into the hydrogel prior to sterilization and sold as a finished device. Exposure of the hydrogel to the solution causes the hydrogel to swell.

As shown in Fig. 1b, typically the device 1 is inserted into the duct or vessel 2 having a region to be treated, such as an occlusion due to a deposition of plaque 5 on the vessel wall tissue 9. The device 1 is moved along the vessel to position the balloon 4 at the occlusion site, as shown in Fig. 1c. The vessel may be, for example, a narrow, tortuous opening through which the catheter is passed by torquing from the distal end. As the balloon is inflated the pressure created by the balloon against the tissue compresses the hydrogel and the drug is quickly and freely released for transfer by active diffusion into the plaque and tissue. The pressure applied to the plaque and tissue by the expansion of the balloon during application of the drug enhances transfer of the drug into the tissue and plaque. This process is referred to here as active diffusion. The balloon and catheter may be exposed to the body fluids of the lumen for a considerable time, e.g., up to about 15 minutes in some angioplasty procedures. An advantage is that large amounts of the drug, e.g., greater than 20%, even 30-50% or more, of the drug solution contained in the hydrogel, is diffused into the affected area in the short time duration which the hydrogel is compressed, e.g., 2-10 minutes after the balloon is inflated at the treatment site. The inflation pressure needed to dilate the vessel which also approximates the compression of the coating, is in the range of 101 to 2027 kPa (1 to 20 atmospheres), typically about 203 to 1013 kPa (about 2 to 10 atmospheres). The balloon is preferably a compliant material such as polyethylene which conforms to the shape of the lumen wall. The balloon may also be formed of other materials used in angioplasty, e.g., a nondistending material, such as polyethylene terephthalate (PET). Transporting the drug in the hydrogel prevents substantial release of the drug to body fluids prior to reaching the treatment area and during the drug application phase and allows large dosages to be delivered at a desired location.

In Fig. 1c, the balloon coating 6 is a swellable, compressible coating formed of the hydrogel and drug in solution. In Fig. 1d, the balloon 4 is shown inflated such that the coating 6, which has an initial thickness, is in contact with the occlusion 5 but not under substantial pressure. Further inflation of the balloon 4, as shown in Fig. 1e, compresses the hydrogel coating 6 against the occluded areas 5 causing quick release of the drug (represented by circles) contained in the coating 6 directly into the plaque and nearby healthy tissue, as indicated by the directional arrows, much in the nature of squeezing liquid from a sponge. The introduction of the drug into the plaque and tissue occurs simultaneously with widening of the occlusion by the dilatation balloon. Thus, as cracking of the plaque and stimulation of smooth muscle cells beneath the plaque and along healthy tissue of the vessel wall are caused by dilatation, a therapeutic drug is simultaneously applied to the affected area, e.g., to counteract the effects of the trauma. The thickness of the balloon 4 remains substantially the same, while the thickness of the coating 6 decreases due to the compression of the coating and the release of the drug 8. (Figs. 1d-1e are schematic drawings and are not to scale with respect to the thickness of the balloon relative to the thickness of the hydrogel coating.) The drug carried by the balloon is evenly applied to plaque and tissue and isolated by the pressure of the balloon from the flow of body fluids in the lumen such that the drug, e.g., an anti-proliferative, may actively diffuse through the cracks formed in the plaque and reach the smooth muscle tissue. (It will also be understood that, as an alternative procedure, after dilation with a conventional angioplasty balloon catheter, a catheter carrying a drug-delivery, inflatable balloon, such as has been described, may be used to treat the vessel.)

The hydrogel coating is characterized by the ability to incorporate a substantial amount of the drug, typically in aqueous solution form, and is swellable such that the aqueous drug solution can be effectively squeezed out of the coating when pressure is applied by inflation of the balloon. Administration of the drug in this way enables the drug to be site specific, such that release of high concentrations and/or highly potent drugs may be limited to direct application to the diseased tissue. Furthermore, the drug is applied to the diseased tissue by the sponge-like coating in an even, gentle manner without disrupting or injuring the healthy tissue, while diffusion of the drug into the tissue is facilitated by the application of the pressure of the inflated balloon. The pressure also effectively forms a seal that prevents the flow of body fluids from washing the drug downstream of the treatment area. The dosage applied to the tissue may be controlled by regulating the time of presoaking the drug into the hydrogel coating to determine the amount of absorption of the drug solution by the hydrogel coating. Other factors affecting the dosage are the concentration of the drug in the solution applied to the coating and the releasability of the hydrogel coating, determined by, for example, the thickness of the hydrogel coating, its resiliency, porosity and the ability of the hydrogel coating to retain the drug, e.g., electrostatic binding or pore size, or the ionic strength of the coating, e.g., changed by changing the pH.

The drug may be an anti-thrombogenic drug, such as heparin or a heparin derivative, Pebac (dextrophenylalanine proline arginine chloromethylketone) or an anti-proliferative, such as heparin (also known to have anti-proliferative properties), enoxaprin, angiopeptin, or monoclonal antibodies capable of blocking smooth muscle cell proliferation, or it may be hirudin or acetylsalicylic acid (i.e., aspirin). Dosages applied to the tissue, for example, of heparin are typically in the range of 10-30 mg of heparin solution containing 200-1,000 units of sodium heparin. For use with hydrogels, the drug is preferably water soluble, so that the drug may be easily absorbed into the coating matrix.

The sponge-like characteristics of the hydrogel allows the aqueous drug solution to be effectively squeezed out of the coating when pressure is applied by inflation of the balloon. The hydrogel and drug combination are preferably noncomplexed, i.e., held together through the ability of the hydrogel to swell and absorb the drug solution, thereby allowing the preferable free-release of the drug at the treatment site.

In particular embodiments it may be advantageous to select a hydrogel coating for a particular drug such that the drug is not substantially released into body fluids prior to application of pressure by expansion of the balloon. Binding of the drug may also be accomplished by electrostatic attraction of the drug to the coating or a coating additive or by mechanical binding, e.g., employing a coating having a pore size that inhibits inward flow of body fluids or outward flow of the drug itself, that might tend to release the drug. Hydrogels are particularly advantageous in that the drug is held within the hydrogen-bond matrix formed by the gel.

The hydrogel is a cross-linked polymer material formed from the combination of a colloid and water. Cross-linking reduces solubility and produces a jelly-like polymer that is characterized by the ability to swell and absorb a substantial amount of the drug, typically in aqueous solution form. The hydrogel coating is also particularly hydrophilic, water swellable, and lubricous (i.e., having a low coefficient of friction). Preferred hydrogels are polyacrylic acid polymers available as HYDROPLUS® (Boston Scientific, Watertown, MA and as described in US Patent No. 5091205, US application no. 297,331, filed January 17, 1991). The drug e.g., heparin in aqueous solution, is absorbed into the coating without complexing and is freely released therefrom. Such hydrogel-drug combinations deliver about half of the drug solution in response to pressures in the range of balloon angioplasty in the vascular system. In other particular embodiments, the hydrogel polymer includes acid groups and incorporates a drug which is anionic in nature that is bound by electrostatic attraction to cations in the coating, such as an ammonium cation, as described in "Lubricous Antithrombogenic Catheters, Guidewires, and Coatings," by Ronald Sahatjian et al., US Patent No. 5135516, USSN 7/451,507 filed on December 15,1989, (see also corresponding International Publication No. WO91/08790, published June 27, 1991). The coating incorporating the quaternary ammonium salt is effective to deliver an initial fast release of drug during compression and a slow release of drug remaining in the compressed coating after compression and is particularly useful for coating vascular stents as described further below.

In general, when dry, the hydrogel coating is preferably on the order of about 1 to 10µm (microns) thick, with a 2 to 5 µm (microns) coating typical. Very thin hydrogel coatings, e.g., of about .2-.3µm (microns) (dry) and much thicker hydrogel coatings, e.g. more than 10µm (microns) (dry) are also possible. Typically, the hydrogel coating thickness may swell by about a factor of 6 to 10 or more when the hydrogel coating is hydrated. For example, a hydrogel coating of about 1 to 3µm (microns) thickness, when dry, usually swells to about 10-30µm (microns) thickness, when hydrated. Most preferably, the thickness of the coating is about 10 to 50µm (microns) in the swelled, uncompressed state, and incorporates about 20-30mg of drug solution.

Referring to Fig. 2, in another embodiment of a drug delivery balloon catheter, the catheter 3 is preferably very small in diameter and flexible, much in the nature of a guidewire, formed, in this case, of hollow tubing to which the coated balloon 4 is attached. The balloon is covered by a protective sheath 7 while the instrument 1 is inserted into the vessel or duct 2 and positioned at the treatment region. As the coated balloon 4 is positioned at the occluded site 5, (Fig. 2a) the protective sheath 7 is drawn back to expose the balloon 4. In an alternative embodiment, the sheath remains stationary while the guidewire-like catheter moves the coated balloon forward into the occluded region. The sheath 7 protects the coating and inhibits premature release of the drug. Such a sheath might be particularly advantageous with coatings and drugs without substantial chemical or mechanical binding. Additionally, the balloon catheter may be a thermal balloon catheter with electrodes 43, as more fully described below. The application of such heat may be employed to facilitate the release of the drug from the coating, to facilitate penetration of the drug into the tissue, or to facilitate the therapeutic action of the drug.

A procedure for preparing a drug delivery balloon with a hydrogel coating and an experiment of drug delivery for the above embodiments are presented in the following examples.

### Examples

### Example 1

A hydrogel coating on an angioplasty balloon may be formed as follows. The surface of the balloon (polyethylene) of an angiographic catheter is prepared by wiping down with clean cloth. The balloon has an O.D. (outer diameter) of about 3.5 mm (inflated). The balloon is coated with a solution of 4,4' diphenylmethane diisocyanate (MDI) in methylethylketone for 30 minutes. After drying in an air oven at 85° C for 30 minutes, the balloon is dipped in a 1.7% solution of poly(acrylic acid) homopolymer having a molecular weight of about 3,000,000 in dimethylformamide (DMF) and tertiarybutyl alcohol. After drying at about 85° C for 30 minutes, a smooth coating is obtained. The balloon is oven dried for 8 hours at 50° C. One function of the drying steps is to remove solvent from the coating. The surface of the balloon becomes instantly lubricous upon exposure to water. The polyisocyanate solution is at a concentration of about .5 to 10% by weight. The polyacrylic acid is at a concentration of about .1 to 10% by weight. The poly(carboxylic acid) to polyisocyanate molar ratio is generally about 1:1. The formation of the hydrogel is further described in US Patent No. 5091205 (USSN 297,331).

A solution of heparin salt may be applied to the coating. The solution is 10,000 units heparin sodium injection (Fisher Scientific, Pittsburgh, PA) USP Grade (1000 units/ml which is then added to 650cc distilled water) and may be applied by dipping for, e.g., about 1 minute at room temperature. The heparin does not form a complex with the hydrogel solution and is freely released in response to compression of the polymer.

After a catheter is prepared for use as discussed above, the catheter may be introduced into the patient using the Seldinger technique and expanded at a desired location to compress the hydrogel and deliver the heparin solution.

### Example 2

Delivery of a drug from a hydrogel coating on a balloon was investigated in the following experiment. Tritium-labeled Pebac was absorbed into a 3.5 mm Slider® (balloon catheter from Boston Scientific Corporation) balloon coated with about a 40µm (micron) thick (in the swelled state) coating as described in Example 1. The coating was dried and the radioactivity was counted. The balloon was then wetted with saline to swell the coating area. The balloon was inflated over a period of about one minute to about 405 kPa (about 4 atmospheres) and held at this pressure for about 10 minutes in a thrombus created in an AV shunt from a baboon. The balloon was withdrawn and the amount of the drug in the thrombus was counted with a radiation counter. The experiment was performed with two different balloons using two different concentrations of Pebac, one balloon with 1-2 mg Pebac, and one balloon with 4mg Pebac. Both balloons delivered about 50% of the Pebac into the thrombus.

### Other Embodiments

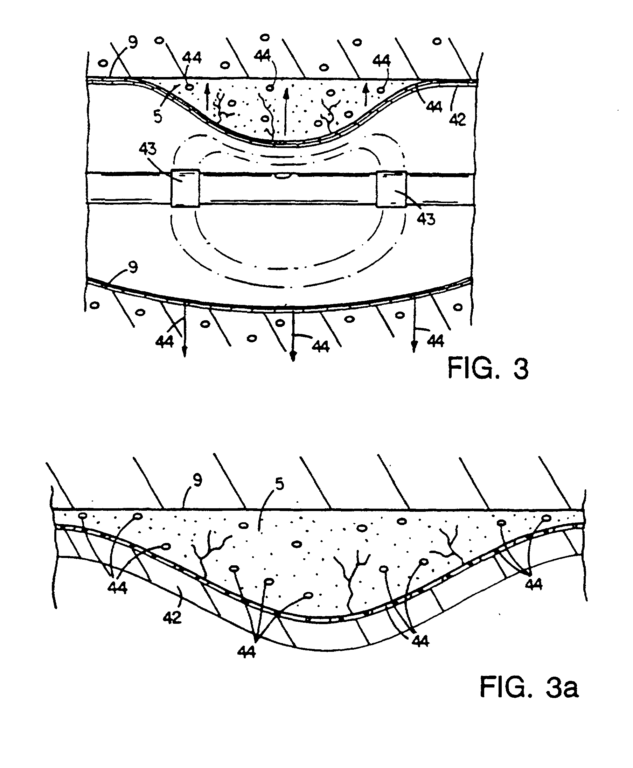

Referring to Fig. 3, in another embodiment, the drug 44 is held within a polymer coating applied to the exterior of a thermal balloon, central wall portions 42 of which are shown in Fig. 3. The balloon is positioned in the lumen in the region to be treated and inflated such that the polymer coating is in contact with the tissue as shown in Figs. 3-3a. Heating of the balloon 42 melts the polymer and releases the drug 44 in a gentle, even, low-energy manner into the affected tissue. Suitable polymers include, but are not limited to, albumin, and collagen, e.g., gelatin, such as gel foams, which typically melt between about 40-60° C or polyvinylpyrrolidone (PVP), which dissolves rapidly when heated. The thermal balloon typically operates between 40-80° C. A suitable heated balloon system for this embodiment or that of Fig. 2a is discussed in Lennox et al., "Heated Balloon Catheters and the Like," U.S. Patent No. 4,955,377 . Inflating liquid is heated as a result of I²R losses by radiofrequency current flowing in the inflation fluid, e.g., saline, between the electrodes 43 (see Figs. 2a and 3), the liquid in turn heating the balloon wall. In the alternative, drugs which melt and which could be applied either with a meltable or non-meltable polymer binder might be used.

An advantage to the meltable coatings is that the polymer may be cross-linked, (e.g., by physical or chemical cross-linking) after application of the drug 44 to the balloon to inhibit release of the drug 44 as the balloon 42 is introduced through the body lumen to the area of treatment. The polymer itself typically does not melt off the balloon, but rather softens in a manner permitting release. However, in embodiments where the polymer is bioabsorbable, e.g., polycaprolactone, polyorthoesters, polylactic acids, and polyglycolic acids, some or even all of the polymer may dissolve off of the balloon.

The balloon may also be coated with a polymer incorporating a drug and inflated to press against the wall of the body lumen, where the polymer is selected to separate from the balloon and coat the wall of the lumen, in response to such pressure with or without the application of heat from the balloon. After application of the polymer, the balloon can be deflated and removed. In this embodiment, the polymer may be a blood soluble polymer such as albumin, collagen or the like, incorporating a drug such as heparin. The polymer produces a smooth coating on the wall of the lumen and releases the drug to the tissue over time as the polymer dissolves. Other soluble polymers are meltable and bioabsorbable polymers discussed above.

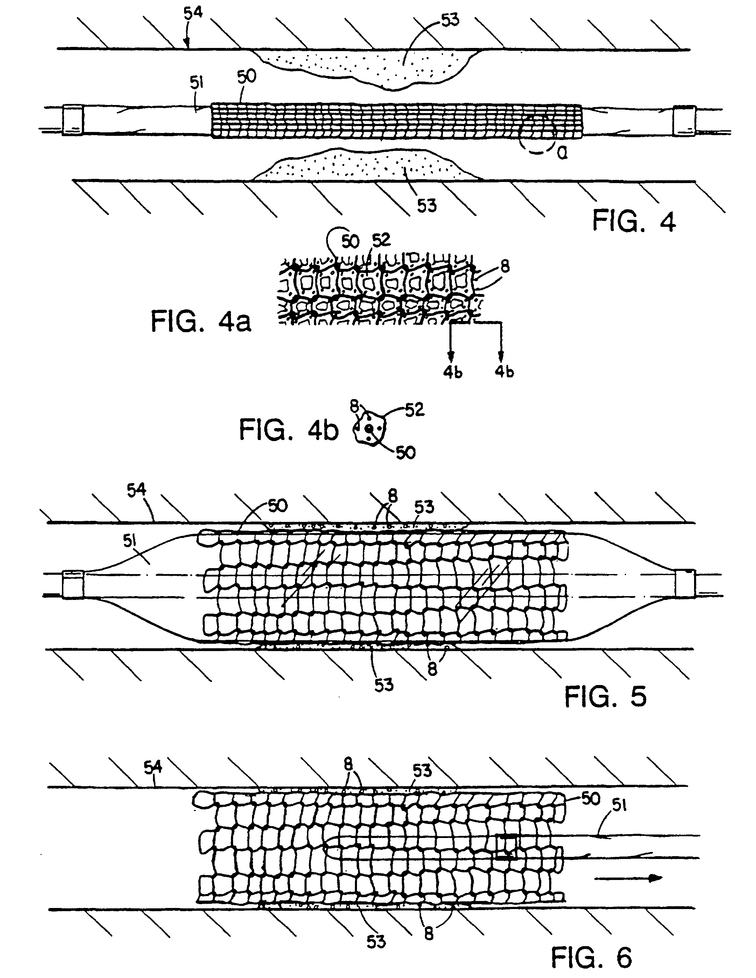

In another embodiment (see Figures 4-6) an endoprosthesis (stent) of the invention is used in combination with a balloon catheter drug delivery system. An endoprosthesis 50 is placed over the balloon catheter 51, and then coated with a noncomplexed hydrogel coating 52. The drug 8, shown as circles, in aqueous solution is then absorbed into the hydrogel coating 52. The balloon 51 and hydrogel and drug coated endoprosthesis 50 are slid until they reach the region of the occlusion 53 in the vessel 54. This is shown in Fig. 4. An enlargement of the drug and hydrogel polymer coated endoprosthesis 50 is shown in Figs. 4a and 4b (thickness of coating 52 is greatly exaggerated). After the balloon 51 and hydrogel and drug coated endoprosthesis 50 have been positioned inside the vessel 54, the endoprosthesis 50 is radially expanded by the admission of pressure to the balloon 51 and compressed against the vessel wall 54 with the result that occlusion 53 is compressed, and the vessel wall 54 surrounding it undergoes a radial expansion. The pressure from inflating the balloon squeezes the hydrogel 52, freely releasing the drug 8 into the tissue. The endoprosthesis 50 is held in position in the expanded state as shown in Fig. 5. The pressure is then released from the balloon and the catheter is withdrawn from the vessel. Figure 6 shows the drug 8 inside the compressed thrombus with the endoprosthesis expanded and left in position, with the balloon catheter being withdrawn from the lumen. It will be understood that only the endoprosthesis may include the hydrogel polymer coating. In the embodiments employing a hydrogel-coated stent, the hydrogel and drug are selected such that an initial high dosage of drug is delivered to adjacent tissue upon initial compression of the polymer and thereafter a slow, sustained time-release of drug remaining in the hydrogel polymer occurs. Preferred hydrogel-drug combinations are those that employ a binding of the drug, such as electrostatic binding, e.g., by using a polyacrylic acid hydrogel in combination with an ammonium cation and heparin. In this case, the coating continues to release drug after expansion of the stent and removal of the balloon catheter. The stent may be a balloon-expansible stent as described above or a self-expanding stent, e.g., of the type formed with superelastic materials such as Nitinol.

Any of the embodiments discussed herein can be used with a protective sheath as described in Figs. 2-2a. In addition, a heated balloon catheter may be used in all combinations of the embodiments above to enhance and control the rate of drug-solution delivery into tissue. other compressible sponge-like polymers, e.g., non hydrogels which release drug solutions in response to pressure, might be used as described with respect to the embodiment of Fig. 1 et seq.

Still other embodiments are within the claims.

## Claims

1. An endoprosthesis (50) comprising a swellable hydrogel polymer coating (52) into which a drug (8) is incorporated.

2. An endoprosthesis according to claim 1, wherein said endoprosthesis (50) is a stent.

3. An endoprosthesis according to claim 1 or claim 2, wherein said endoprosthesis (50) is mounted on a catheter comprising a balloon (51).

4. An endoprosthesis according to any one of the preceding claims, wherein the endoprosthesis (50) is adapted to deliver a high dosage of drug (8) to tissue of a body lumen (54) upon initial application of pressure to said endoprosthesis (50) and said polymer (52) against said lumen (54) followed by a slow, sustained release of said drug (8) from said polymer coating (52) after the application of pressure ceases.

5. An endoprosthesis according to claim 4, wherein said initial application of pressure is in the range of 101 to 2027 kPa (1 to 20 atmospheres).

6. An endoprosthesis according to claim 4 or claim 5, wherein the endoprosthesis is adapted to deliver a high dosage of drug to a duct.

7. An endoprosthesis according to claim 4 or claim 5, wherein the endoprosthesis is adapted to deliver a high dosage of drug to a vessel.

8. An endoprosthesis according to any one of the preceding claims wherein said hydrogel polymer is selected from the group consisting of polycarboxylic acids, cellulosic polymers, gelatin, polyvinylpyrrolidone, maleic anhydride polymers, polyamides, polyvinyl alcohols, and polyethylene oxides.

9. An endoprosthesis according to claim 8, wherein said hydrogel polymer is polyacrylic acid.

10. An endoprosthesis according to claim 3, wherein said hydrogel polymer coating (52) is applied to the exterior of the balloon (51).

11. An endoprosthesis according to claim 10, wherein said polymer is a meltable polymer which is gelatin or polyvinylpyrrolidone.

12. An endoprosthesis according to any one of the preceding claims, wherein said drug is electrostatically bound to said polymer coating, wherein the polymer coating includes acid groups and incorporates said drug which is anionic in nature.

13. An endoprosthesis according to any one of the preceding claims wherein said drug is an anti-thrombogenic drug selected from the group consisting of heparin, Pebac, enoxaprin, aspirin and hirudin.

14. An endoprosthesis according to any one of claims 1 to 12, wherein said drug is an anti-proliferative drug selected from monoclonal antibodies capable of blocking smooth muscle cell proliferation, heparin, angiopeptin and enoxaprin.

## Patentansprüche

1. Endoprothese (50), umfassend eine quellbare Hydrogelpolymerbeschichtung (52), in welche ein Arzneistoff (8) eingebracht ist.

2. Endoprothese gemäß Anspruch 1, wobei die Endoprothese (50) ein Stent ist.

3. Endoprothese gemäß Anspruch 1 oder Anspruch 2, wobei die Endoprothese (50) an einem Katheter angebracht wird, der einen Ballon (51) umfasst.

4. Endoprothese gemäß einem der vorangegangenen Ansprüche, wobei die Endoprothese (50) angepasst ist, eine hohe Dosierung eines Arzneistoffs (8) an Gewebe eines Körperlumens (54) nach Anfangsanwendung von Druck auf die Endoprothese (50) und des Polymers (52) gegen das Lumen, gefolgt von einer langsamen, verlängerten Freisetzung des Arzneistoffs (8) aus der Polymerbeschichtung (52), nachdem die Anwendung von Druck aufgehört hat, abzugeben.

5. Endoprothese gemäß Anspruch 4, wobei die Anfangsanwendung von Druck im Bereich von 101 bis 2027 kPa (1 bis 20 Atmosphären) liegt.

6. Endoprothese gemäß Anspruch 4 oder Anspruch 5, wobei die Endoprothese angepasst ist, eine hohe Dosierung eines Arzneistoffs an eine Leitung abzugeben.

7. Endoprothese gemäß Anspruch 4 oder Anspruch 5, wobei die Endoprothese angepasst ist, eine hohe Dosierung eines Arzneistoffs an ein Gefäß abzugeben.

8. Endoprothese gemäß einem der vorangegangenen Ansprüche, wobei das Hydrogelpolymer ausgewählt ist aus der Gruppe, bestehend aus Polycarbonsäuren, Cellulosepolymeren, Gelatine, Polyvinylpyrrolidon, Maleinsäureanhydridpolymeren, Polyamiden, Polyvinylalkoholen und Polyethylenoxiden.

9. Endoprothese gemäß Anspruch 8, wobei das Hydrogelpolymer Polyacrylsäure ist.

10. Endoprothese gemäß Anspruch 3, wobei die Hydrogelpolymerbeschichtung (52) auf die Außenseite des Ballons (51) aufgebracht ist.

11. Endoprothese gemäß Anspruch 10, wobei das Polymer ein schmelzbares Polymer ist, welches Gelatine oder Polyvinylpyrrolidon ist.

12. Endoprothese gemäß einem der vorangegangenen Ansprüche, wobei der Arzneistoff an der Polymerbeschichtung elektrostatisch gebunden ist, wobei die Polymerbeschichtung Säurereste einschließt und den Arzneistoff einbaut, welcher dem Wesen nach anionisch ist.

13. Endoprothese gemäß einem der vorangegangenen Ansprüche, wobei der Arzneistoff ein antithrombogener Arzneistoff ist, der ausgewählt ist aus der Gruppe, bestehend aus Heparin, Pebac, Enoxaprin, Aspirin und Hirudin.

14. Endoprothese gemäß einem der Ansprüche 1 bis 12, wobei der Arzneistoff ein antiproliferativer Arzneistoff ist, der ausgewählt ist aus der Gruppe, bestehend aus monoklonalen Antikörpern, die in der Lage sind, die Proliferation glatter Muskelzellen zu blockieren, Heparin, Angiopeptin und Enoxaprin.

## Revendications

1. Endoprothèse (50) comprenant un revêtement en polymère d'hydrogel gonflable (52) dans lequel un médicament (8) est incorporé.

2. Endoprothèse selon la revendication 1, dans laquelle ladite endoprothèse (50) est une endoprothèse vasculaire.

3. Endoprothèse selon la revendication 1 ou la revendication 2, dans laquelle ladite endoprothèse (50) est montée sur un cathéter comprenant un ballon (51).

4. Endoprothèse selon l'une quelconque des revendications précédentes, dans laquelle l'endoprothèse (50) est adaptée à fournir un dosage élevé de médicament (8) à un tissu d'une lumière corporelle (54) lors de l'application initiale de pression sur ladite endoprothèse (50) et ledit polymère (52) contre ladite lumière (54) suivie par une libération lente prolongée dudit médicament (8) depuis ledit revêtement en polymère (52) après la fin de l'application de pression.

5. Endoprothèse selon la revendication 4, dans laquelle ladite application initiale de pression se trouve dans la plage comprise entre 101 et 2027 kPa (1 à 20 atmosphères).

6. Endoprothèse selon la revendication 4 ou la revendication 5, dans laquelle l'endoprothèse est adaptée à fournir un dosage élevé de médicament à un conduit.

7. Endoprothèse selon la revendication 4 ou la revendication 5, dans laquelle l'endoprothèse est adaptée à fournir un dosage élevé de médicament à un vaisseau.

8. Endoprothèse selon l'une quelconque des revendications précédentes, dans laquelle ledit polymère d'hydrogel est sélectionné parmi le groupe constitué des acides polycarboxyliques, des polymères cellulosiques, de la gélatine, de la polyvinylpyrrolidone, des polymères d'anhydride maléique, des polyamides, des alcools polyvinyliques et des oxydes de polyéthylène.

9. Endoprothèse selon la revendication 8, dans laquelle ledit polymère d'hydrogel est l'acide polyacrylique.

10. Endoprothèse selon la revendication 3, dans laquelle ledit revêtement en polymère d'hydrogel (52) est appliqué à l'extérieur du ballon (51).

11. Endoprothèse selon la revendication 10, dans laquelle ledit polymère est un polymère qui peut fondre qui est la gélatine ou la polyvinylpyrrolidone.

12. Endoprothèse selon l'une quelconque des revendications précédentes, dans laquelle ledit médicament est lié de manière électrostatique audit revêtement en polymère, dans laquelle le revêtement en polymère comporte des groupes acides et incorpore ledit médicament qui est anionique de nature.

13. Endoprothèse selon l'une quelconque des revendications précédentes, dans laquelle ledit médicament est un médicament anti-thrombogénique sélectionné parmi le groupe constitué de l'héparine, du Pebac, de l'énoxaprine, de l'aspirine et de l'hirudine.

14. Endoprothèse selon l'une quelconque des revendications 1 à 12, dans laquelle ledit médicament est un médicament anti-prolifératif sélectionné parmi des anticorps monoclonaux capables de bloquer la prolifération de cellules musculaires lisses, l'héparine, l'angiopeptine et l'énoxaprine.
